# EUROPÄISCHE PATENTSCHRIFT

(11) **EP 3 558 912 B1**
(45) Veröffentlichungstag und Bekanntmachung des Hinweises auf die Patenterteilung: **02.12.2020**
(21) Anmeldenummer: 17825248.2
(22) Anmeldetag: 22.12.2017
(51) Int. Cl.: C07C 5/48

(54) **VERFAHREN UND ANLAGE ZUR HERSTELLUNG EINES OLEFINS**
METHOD AND ASSEMBLY FOR MANUFACTURING AN OLEFIN
PROCÉDÉ ET INSTALLATION DE PRODUCTION D'UNE OLÉFINE

(30) Priorität: 22.12.2016 EP 16206476
(43) Veröffentlichungstag der Anmeldung: 30.10.2019
(73) Patentinhaber: Linde GmbH, 82049 Pullach (DE)
(72) Erfinder: PESCHEL, Andreas, 82515 Wolfratshausen (DE); OBERMEIER, Andreas, 85658 Egmating (DE); FRITZ, Helmut, 81375 München (DE); ZELLHUBER, Mathieu, 82152 Martinsried (DE)
(74) Vertreter: m patent group
(86) Internationale Anmeldenummer: PCT/EP2017/084502
(87) Internationale Veröffentlichungsnummer: WO 2018/115494

(56) Entgegenhaltungen:
- EP-A1- 3 029 402
- DE-A1-102012 020 470

## Beschreibung

Die Erfindung betrifft ein Verfahren zur Herstellung eines Olefins und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die Bildungsenergien ΔG für Ethan, Propan bzw. n-Butan betragen -102, -115 bzw. -118 kJ/mol. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und Lopez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767 bis 834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen. EP3029402 betrifft ein Verfahren zur trenntechnischen Bearbeitung eines an Wasserstoff, Methan und Kohlenwasserstoffen mit zwei Kohlenstoffatomen reichen Einsatzstroms und eine entsprechende Anlage.

Die Erfindung wird nachfolgend insbesondere unter Bezugnahme auf die ODH von Ethan (sogenannte ODH-E) beschrieben. Ihr Einsatz ist jedoch grundsätzlich auch bei der ODH höherer Paraffine wie Propan und Butan möglich und vorteilhaft. Neben der oxidativen Dehydrierung von Ethan kann prinzipiell auch eine nichtoxidative Dehydrierung von Ethan zur Herstellung von Ethylen erfolgen. Auch für ein derartiges Verfahren eignet sich die vorliegende Erfindung.

Für die nachhaltige Aktivität der Katalysatoren in der ODH ist ein Mindestmaß an Sauerstoff am Reaktoraustritt erforderlich, um eine Reduktion des Katalysators und damit einen Verlust in dessen Leistungsfähigkeit zu vermeiden. Aus diesem Grund kann in der Regel nicht mit einem vollständigen Sauerstoffumsatz im Reaktor gearbeitet werden. Des Weiteren werden bei höheren Umsätzen nennenswerte Mengen an Kohlenmonoxid und Kohlendioxid und ggf. Carbonsäuren als Nebenprodukte gebildet. Methan kann ebenfalls als Nebenprodukt gebildet werden oder bereits im Einsatz in den Reaktor enthalten sein und den Reaktor als sich inert verhaltende Komponente im Wesentlichen unbeeinflusst durchlaufen. Die genannten Komponenten in einem entsprechenden Prozessgas, d.h. dem dem Gasgemisch aus dem Reaktor, müssen in nachgeschalteten Trennschritten abgetrennt werden.

Wie auch nachfolgend erläutert, ist es insbesondere aufgrund der geringeren Gehalte an Methan in einem Prozessgas der ODH-E nicht ohne weiteres möglich, bekannte Trennverfahren und Trenneinrichtungen, wie sie beispielsweise für die Trennung von Prozessgasen aus Steamcrackern verwendet werden, einzusetzen, ohne Produkt- und ggf. Eduktverluste in Kauf zu nehmen. Dies betrifft insbesondere einen Trennschritt, in dem Methan und tiefer siedende Komponenten von höher siedenden Komponenten abgetrennt werden, also eine sogenannte Demethanisierung bzw. einen entsprechenden Demethanizer. Wie ebenfalls nachfolgend erläutert, kann es in einer entsprechenden Trennung außerdem dazu kommen, dass sich insbesondere Kohlenmonoxid und Sauerstoff über unerwünschte und bedenkliche Konzentrationen hinaus anreichern. Entsprechendes gilt auch für Prozessgase aus anderen Dehydrierungen und der ODH höherer Paraffine wie Propan und Butan, in denen ebenfalls nur ein relativ geringer Anteil an Verbindungen enthalten ist, die tiefer als die jeweils eingesetzten Edukte und die gebildeten Produkte sieden.

Die vorliegende Erfindung stellt sich die Aufgabe, entsprechende Verfahren und Anlagen zu verbessern und die genannten Probleme in einer entsprechenden Trennung, insbesondere für ein Prozessgas aus der ODH, und weiter insbesondere ein Prozessgas aus der ODH-E, zu adressieren.

### Offenbarung der Erfindung

Vor diesem Hintergrund schlägt die vorliegende Erfindung ein Verfahren zur Herstellung eines Olefins und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 99%, 99,5%, 99,9% oder 99,99% und die Angabe "arm" für einen Gehalt von höchstens 1%, 0,5%, 0,1% oder 0,01% auf molarer, Gewichts-oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Methan" die Rede, kann es sich um ein Reingas, aber auch ein an der jeweiligen Komponente reiches Gemisch handeln. Ein Gasgemisch, das "überwiegend" eine oder mehrere Komponenten enthält, ist insbesondere reich an dieser oder diesen im erläuterten Sinn.

Stoffströme, Gasgemische usw. können im hier verwendeten Sprachgebrauch außerdem "angereichert" oder "abgereichert" an einer oder mehreren Komponenten sein, wobei sich diese Begriffe auf einen Gehalt in einem Ausgangsgemisch beziehen. Sie sind "angereichert", wenn sie zumindest den 1,5-fachen, 2-fachen, 5-fachen, 10-fachen, 100-fachen oder 1.000-fachen Gehalt, "abgereichert", wenn sie höchstens den 0,75-fachen, 0,5-fachen, 0,1-fachen, 0,01-fachen oder 0,001-fachen Gehalt einer oder mehrerer Komponenten, bezogen auf das Ausgangsgemisch, enthalten.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil der Sumpfflüssigkeit verdampft und gasförmig in die Rektifikationskolonne zurückgespeist.

Die vorliegende Erfindung betrifft Trennverfahren und entsprechende Trenneinrichtungen, die dem grundlegenden Konzept nach, nicht jedoch in der erfindungsgemäß realisierten Ausführung, Trennverfahren und Trenneinrichtungen ähneln, wie sie für andere Prozessgase bekannt sind, beispielsweise Prozessgase aus Steamcrackern. Diese sind in der Fachliteratur, beispielsweise im Artikel "Ethylene" in Ullmann's Encyclopedia of Industrial Chemistry, Onlineausgabe, 15. April 2007, DOI 10.1002/14356007.a10_045.pub2, beschrieben. Ein wesentlicher Schritt in derartigen Trennverfahren ist häufig die sogenannte Demethanisierung, in der aus dem Prozessgas, ggf. bereits nach Abtrennung weiterer Komponenten, Methan und tiefer als Methan siedende Verbindungen von höher siedenden Komponenten abgetrennt werden. Zu Details bekannter Demethanisierungsverfahren sei auf die zitierte Fachliteratur verwiesen.

### Vorteile der Erfindung

Wie eingangs erwähnt, sind in einem typischen, mittels ODH gebildeten Prozessgas neben Hauptprodukten wie Olefinen (und typischerweise Carbonsäuren) unter anderem nicht umgesetzte Paraffine, Methan, Sauerstoff, Kohlenmonoxid und Kohlendioxid enthalten. Das Prozessgas enthält typischerweise auch Wasser sowie ggf. geringere Mengen Inertgase, wobei unter "Inertgasen" hier allgemein solche Gase verstanden werden, die in der ODH nicht oder allenfalls zu geringen Anteilen reagieren, und nicht nur die klassischen Inertgase wie Stickstoff oder Edelgase. Auch Methan verhält sich in der ODH-E beispielsweise im Wesentlichen inert.

Entsprechendes gilt in vergleichbarer Weise für Prozessgase aus anderen Verfahren zur Herstellung von Olefinen, beispielsweise der Dehydrierung von Ethan, wobei nachfolgend, wie mehrfach erwähnt, vereinfachend auf die ODH und insbesondere auf die ODH-E Bezug genommen wird. In einer der ODH nachgeschalteten Trennung müssen die genannten Komponenten abgetrennt werden.

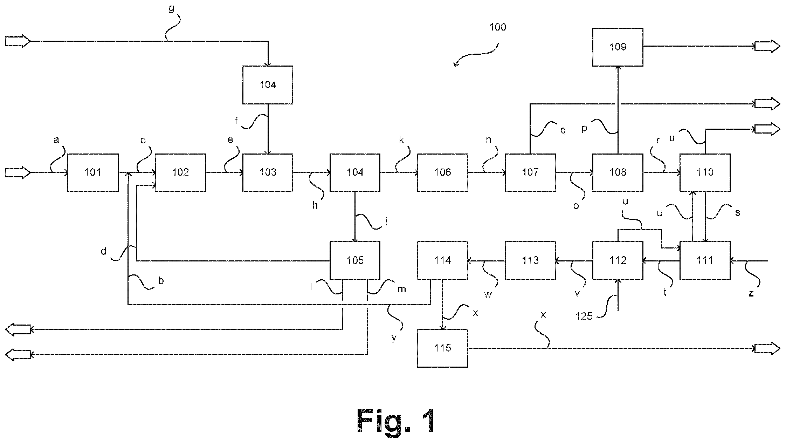

Die Trennung erfolgt, wie auch unter Bezugnahme auf die beigefügte Figur 1 veranschaulicht, typischerweise nach einer Verdichtung des Prozessgases. Dieses wird anschließend typischerweise mittels einer Wasserwäsche zumindest von großen Teilen der ggf. vorhandenen Carbonsäuren und des enthaltenen Wassers befreit. Anschließend erfolgen Waschschritte zur Entfernung von Kohlendioxid.

Das nun noch im Wesentlichen Olefine, die nicht umgesetzten Paraffine, Sauerstoff, Kohlenmonoxid sowie geringere Mengen Inertgase enthaltende Prozessgas wird von Restwasser befreit und vorgekühlt. In einer sich anschließenden Tieftemperaturtrennung erfolgt nun eine stufenweise Kondensation des Prozessgases. Die verbleibenden Gasfraktionen werden jeweils dem nächsten Kondensationsschritt zugeführt. Die Kondensate werden in einer Tieftemperaturrektifikation getrennt, wobei wiederum eine Gasfraktion sowie eine flüssige Fraktion gebildet werden. Die Gasfraktion aus der Tieftemperaturrektifikation wird typischerweise mit der stromab des letzten Kondensationsschritts verbleibenden Gasfraktion zu einer weiteren Gasfraktion, der sogenannten Brenngasfraktion bzw. Tailgasfraktion, vereinigt und typischerweise nach einer wärmeintegrierten Verwendung als Kältemittel einer thermischen Nutzung zugeführt. Die flüssige Fraktion der Tieftemperaturrektifikation wird weiteren Trennschritten unterworfen.

Die Brenngasfraktion soll dabei zumindest den überwiegenden Teil des in dem Prozessgas, das der Tieftemperaturtrennung zugeführt wurde, enthaltenen Kohlenmonoxids und Sauerstoffs sowie der übrigen, tiefer als die gewünschten Olefine siedenden Komponenten enthalten. Die gewünschten Olefine sollen zumindest überwiegend in die flüssige Fraktion aus der Tieftemperaturrektifikation übergehen, gemeinsam mit den höher siedenden, nicht umgesetzten Paraffinen.

Die Trenneffizienz in der Tieftemperaturrektifikation richtet sich wesentlich nach dem Gehalt leichter Komponenten in dem Prozessgas, das der Tieftemperaturrektifikation zugeführt wird, weil hierbei ein flüssiger Rücklauf verwendet wird, der im Wesentlichen aus entsprechenden leichten Komponenten gebildet wird. Im Falle der ODH-E handelt es sich hierbei um Methan. Bei zu geringen Gehalten entsprechender leichter Komponenten kann der Rücklauf nicht in ausreichender Menge bereitgestellt werden. Daher ergeben sich bei zu geringen Mengen leichter Verbindungen beträchtliche Verluste an Wertprodukten, insbesondere der gewünschten Olefine, im Fall der ODH-E Ethylen, aber auch ggf. von nicht umgesetzten Edukten, im Fall der ODH-E Ethan. Diese gehen in die Tailgasfraktion über, wodurch wirtschaftliche Nachteile gegenüber anderen Verfahren entstehen.

In der Tieftemperaturtrennung reichern sich ferner die jeweils gebildeten Gasgemische zunehmend mit Sauerstoff und Kohlenmonoxid an. In derartigen Gasgemischen können sowohl die Explosionsgrenze als auch die Sauerstoffgrenzkonzentration (siehe sogleich) deutlich überschritten werden. Allgemein kann bereits ein Sauerstoffanteil von 3000 vppm in einem typischen Prozessgas einer ODH-E am Reaktoraustritt, d.h. im Prozessgas direkt stromab des oder der für die ODH-E verwendeten Reaktoren, dazu führen, dass ein in der Tieftemperaturrektifikation gebildetes Gasgemisch Kohlenmonoxid- und Sauerstoffanteile von 66 Vol.-% bzw. 13 Vol.-% aufweist.

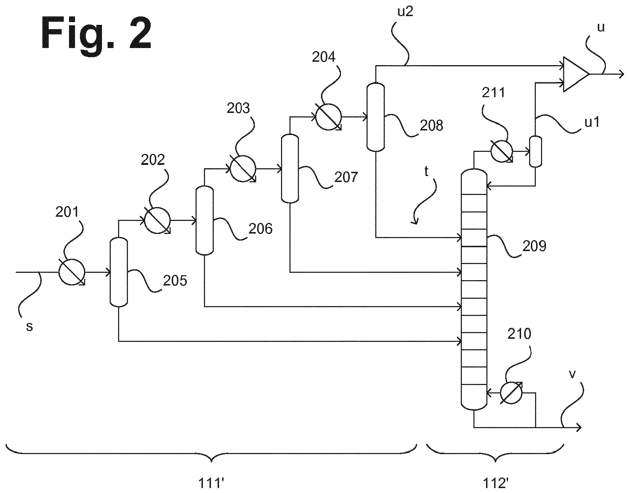

Die (untere) Explosionsgrenze eines Gases gibt den Gehalt in einem Gasgemisch an, ab dem eine Entzündung bzw. Explosion bei zugleich ausreichendem Sauerstoffgehalt möglich ist. Diese liegt für Kohlenmonoxid bei einem Gehalt von deutlich über 10 Molprozent und wird unter anderem durch den Gehalt an Wasserstoff und Wasser in dem jeweils betrachteten Gasgemisch beeinflusst. Unter Standardbedingungen wird sie mit 12,5 Molprozent angegeben. In den erläuterten, in einer typischen Tieftemperaturtrennung stromab einer ODH-E gebildeten Gasfraktion liegt der Gehalt an Kohlenmonoxid bei mindestens 40 und bis zu 70 Molprozent, wie auch unter Bezugnahme auf die beigefügte Figur 2 erläutert und soeben erwähnt, so dass die Explosionsgrenze deutlich überschritten ist.

Wenn die Explosionsgrenze überschritten ist, kann eine Explosion erfolgen, sofern auch die sogenannte Sauerstoffgrenzkonzentration überschritten ist. Diese gibt den Sauerstoffgehalt an, ab dem eine Explosion eintreten kann. Mit anderen Worten müssen, damit eine Explosion möglich ist, sowohl die Explosionsgrenze als auch die Sauerstoffgrenzkonzentration überschritten sein.

Die Sauerstoffgrenzkonzentration für Kohlenmonoxid ist nun ausgesprochen gering und liegt bei etwa der Hälfte von jener von Methan und etwa bei jener von Ethylen. In Gemischen aus unterschiedlichen brennbaren Gaskomponenten stellen sich typischerweise Zwischenwerte ein. Im vorliegenden Fall, d.h. den in der Tieftemperaturtrennung gebildeten Gasgemischen, liegt die Sauerstoffgrenzkonzentration bei einem Wert von ca. 6 Molprozent, sofern keine weiteren Maßnahmen ergriffen werden. Es kann daher grundsätzlich zu Explosionen und ggf. sogar zu Detonationen kommen.

Die vorliegende Erfindung löst diese Probleme in einem Verfahren zur Herstellung eines Olefins mit N Kohlenstoffatomen, bei dem unter Einsatz einer Dehydrierung ein Prozessgas gebildet wird, das zumindest das Olefin mit N Kohlenstoffatomen, ein Paraffin mit N Kohlenstoffatomen und einen Kohlenwasserstoff mit N - 1 Kohlenstoffatomen sowie insbesondere Kohlenmonoxid und Sauerstoff enthält, und bei dem unter Verwendung zumindest eines Teils des Prozessgases ein Trenneinsatz gebildet wird, der einer ersten Tieftemperaturtrennung unterworfen wird, in welcher der Trenneinsatz über mehrere Temperaturniveaus stufenweise abgekühlt und Kondensate aus dem Trenneinsatz abgeschieden werden, wobei die Kondensate zumindest zum Teil unter Erhalt einer ersten Gasfraktion und einer ersten Flüssigfraktion einer ersten Tieftemperaturrektifikation unterworfen werden, wobei die erste Gasfraktion zumindest das Olefin mit N Kohlenstoffatomen in einem geringeren Anteil als in den Kondensaten und den Kohlenwasserstoff mit N - 1 Kohlenstoffatomen in einem höheren Anteil als in den Kondensaten enthält.

Erfindungsgemäß ist vorgesehen, dass die erste Gasfraktion zumindest zum Teil einer zweiten Tieftemperaturrektifikation unter Verwendung eines flüssigen Rücklaufs, der überwiegend oder ausschließlich den Kohlenwasserstoff mit N - 1 Kohlenstoffatomen enthält, unterworfen wird, in der die erste Gasfraktion an dem Olefin mit N Kohlenstoffatomen abgereichert wird. Im Rahmen der vorliegenden Erfindung ist also vorgesehen, zusätzlich zur Verwendung einer in einer entsprechenden Tieftemperaturtrennung üblicherweise verwendeten ("ersten") Tieftemperaturrektifikation eine weitere ("zweite") Tieftemperaturrektifikation einzusetzen, in der die in die Kopffraktion der ersten Tieftemperaturrektifikation, also die "erste Gasfraktion" übergegangenen Wertprodukte und ggf. Edukte zurückgewonnen werden können. Dies kann mittels einer relativ kleinen Rektifikationskolonne erzielt werden, so dass sich der zusätzliche apparative Aufwand in Grenzen hält und durch die deutlich geringeren Produktverluste mehr als kompensiert werden kann.

Die zweite Tieftemperaturrektifikation wird unter Verwendung eines extern bereitgestellten flüssigen Rücklaufs durchgeführt, so dass die Trennleistung unabhängig von den in dem Prozessgas enthaltenen leichten Komponenten ist. Die Bereitstellung eines entsprechenden flüssigen Rücklaufs kann durch das Vorhandensein der jeweils erforderlichen Medien in einer entsprechenden Anlage einfach und kostengünstig bewerkstelligt werden.

Ein bei der stufenweisen Abkühlung gasförmig verbleibender Anteil des Trenneinsatzes, also eine nicht in Form der Kondensate abgeschiedene leichtere Fraktion, kann ebenfalls zumindest teilweise der zweiten Tieftemperaturrektifikation unterworfen werden. Auf diese Weise können auch in diesem Anteil enthaltene Olefine mit N Kohlenstoffatomen als Produkt erhalten werden.

Vorteilhafterweise wird in der zweiten Tieftemperaturrektifikation eine zweite Gasfraktion, die arm an oder frei von dem Olefin mit N Kohlenstoffatomen ist, und eine zweite Flüssigfraktion, die das Olefin mit N Kohlenstoffatomen in einem höheren Anteil als in der ersten Gasfraktion enthält, gebildet. Die zweite Flüssigfraktion kann insbesondere in die erste Tieftemperaturrektifikation zurückgeführt werden, so dass das darin enthaltene Olefin mit N Kohlenstoffatomen letztlich in die erste flüssige Fraktion und damit in eine Produktfraktion übergehen kann.

Der flüssige Rücklauf kann bereits in flüssigem Zustand, d.h. als verflüssigtes Gas, bereitgestellt werden. Beispielsweise kann in der ODH-E, in der N zwei beträgt, der überwiegend oder ausschließlich den Kohlenwasserstoff mit N - 1 Kohlenstoffatomen enthaltende Rücklauf also überwiegend oder ausschließlich Methan enthält, auf Flüssigerdgas zurückgegriffen werden. Auch für andere Werte für N ist Entsprechendes möglich, beispielsweise die Verwendung von flüssigem Ethan, Propan, Butan usw., die beispielsweise in Tanks bereitgestellt werden können.

Mit besonderem Vorteil wird der flüssige Rücklauf unter Verwendung einer flüssigen Fraktion gebildet, die durch Teilkondensieren eines überwiegend oder ausschließlich den Kohlenwasserstoff mit N - 1 Kohlenstoffatomen enthaltenden Gases gebildet wird. Die Teilkondensation kann insbesondere ein Abkühlen eines entsprechenden druckbeaufschlagten Gases und dessen anschließende Entspannung umfassen.

Ein bei dem Teilkondensieren des überwiegend oder ausschließlich den Kohlenwasserstoff mit N - 1 Kohlenstoffatomen enthaltenden Gases gasförmig verbleibender Anteil kann zumindest zum Teil in die Tieftemperaturtrennung eingespeist werden. Dieser Anteil wird beispielsweise in die erste Tieftemperaturrektifikation eingespeist und/oder zumindest zum Teil nach dem Abscheiden wenigstens eines der Kondensate aus dem Trenneinsatz zu dem Trenneinsatz zugegeben. Auf diese Weise kann durch eine entsprechende Verdünnung eine Reduktion des Sauerstoffgehaltes in entsprechenden Bereichen bzw. Fraktionen erzielt werden, so dass sich ein Doppelnutzen der gemäß dieser Ausgestaltung vorgeschlagenen Maßnahmen ergibt. Die Einspeisung kann dabei grundsätzlich an jeder Stelle erfolgen, an welcher eine Reduktion des Sauerstoffgehalts aus Sicherheitsgründen sinnvoll ist, insbesondere in Abscheider, in denen die erwähnten Kondensate abgeschieden werden, in eine in der ersten Tieftemperaturrektifikation eingesetzte Rektifikationskolonne oder in die jeweiligen Leitungen. Die Einspeisung erfolgt insbesondere in einer Menge, die den Sauerstoffgehalt auf einen Wert von unter 6 Molprozent oder 5 Molprozent verringert.

Auch das verflüssigte Gas oder die flüssige Fraktion kann - neben der Verwendung als flüssiger Rücklauf - teilweise entsprechend verwendet werden. Die Einspeisemöglichkeiten sind dabei dieselben wie für den gasförmigen Anteil erläutert. Auch durch die flüssige Einspeisung kann grundsätzlich eine Verdünnung herbeigeführt werden. Durch die Einspeisung des verflüssigten Gases in flüssigem Zustand kann jedoch zusätzlich insbesondere für die Abkühlung benötigte Energie bzw. Wärmetauscherfläche eingespart werden.

Wie erwähnt, kann das erfindungsgemäße Verfahren insbesondere für oxidative oder nichtoxidative Dehydrierungen von Ethan zum Einsatz kommen, also in Verfahren, in denen N zwei ist, so dass das Olefin mit N Kohlenstoffatomen Ethylen, das Paraffin mit N Kohlenstoffatomen Ethan und der Kohlenwasserstoff mit N - 1 Kohlenstoffatomen Methan ist. Insbesondere eignet sich das Verfahren für eine oxidative Dehydrierung, also insbesondere für eine ODH-E. In anderen Fällen kann N auch drei oder vier betragen.

In Fällen, in denen N zwei ist, umfassen die Temperaturniveaus, auf die der Trenneinsatz stufenweise abgekühlt wird, vorteilhafterweise ein erstes Temperaturniveau von -20 bis -40 °C und/oder ein zweites Temperaturniveau von -40 bis -60 °C und/oder ein drittes Temperaturniveau von -70 bis -80 °C und/oder ein viertes Temperaturniveau von -95 bis -105 °C. Derartige Temperaturniveaus lassen sich mit bekannten Kältemittelkreisläufen, beispielsweise Propylen- und Ethylenkältemittelkreisläufen auf unterschiedlichen Drücken, erreichen. Die erste Tieftemperaturrektifikation umfasst vorteilhafterweise eine Kondensation von Kopfgas auf einem Temperaturniveau von -90 bis -100 °C.

In Fällen, in denen N zwei ist, der flüssige Rücklauf also überwiegend oder ausschließlich Methan enthält, wird dieser vorteilhafterweise in Form von verflüssigtem Erdgas bereitgestellt oder unter Verwendung zumindest eines Teils einer flüssigen Fraktion gebildet, die durch Abkühlen eines überwiegend oder ausschließlich Methan enthaltenden Gases auf einem Druckniveau von 30 bis 70 bar auf ein Temperaturniveau von -70 bis -100 °C und Entspannen auf ein Druckniveau von 10 bis 20 bar bereitgestellt wird. Auf diese Weise lassen sich ggf. vorhandene Methanquellen wie beispielsweise Pipelinemethan auf entsprechend hohen Druckniveaus nutzen, ohne dass eine weitere Verdichtung erforderlich ist.

Wie erwähnt, kann das Verfahren insbesondere in der zweiten Tieftemperaturrektifikation vergleichsweise kleine und kostengünstige Rektifikationskolonnen verwenden, beispielsweise solche mit 1 bis 10 theoretischen oder praktischen Böden.

Wie erwähnt, enthält das Prozessgas insbesondere auch Sauerstoff und Kohlenmonoxid, so dass die erfindungsgemäß ebenfalls möglichen Verdünnungsmöglichkeiten zum Explosions- und Detonationsschutz besonders vorteilhaft sind.

Die vorliegende Erfindung erstreckt sich auch auf eine Anlage zur Herstellung eines Olefins mit N Kohlenstoffatomen, mit wenigstens einer Reaktoreinheit, die dafür eingerichtet ist, unter Einsatz einer Dehydrierung ein Prozessgas zu bilden, das zumindest das Olefin mit N Kohlenstoffatomen, ein Paraffin mit N Kohlenstoffatomen und einen Kohlenwasserstoff mit N - 1 Kohlenstoffatomen enthält, und mit Mitteln, die dafür eingerichtet sind, unter Verwendung zumindest eines Teils des Prozessgases ein Trenneinsatz zu bilden und einer ersten Tieftemperaturtrennung zu unterwerfen, die dafür eingerichtet ist, den Trenneinsatz über mehrere Temperaturniveaus stufenweise abzukühlen und Kondensate aus dem Trenneinsatz abzuscheiden sowie die Kondensate zumindest zum Teil unter Erhalt einer ersten Gasfraktion und einer ersten Flüssigfraktion einer ersten Tieftemperaturrektifikation zu unterwerfen, wobei die erste Gasfraktion zumindest das Olefin mit N Kohlenstoffatomen in einem geringeren Anteil als in den Kondensaten und den Kohlenwasserstoff mit N - 1 Kohlenstoffatomen in einem höheren Anteil als in den Kondensaten enthält.

Erfindungsgemäß weist die Anlage Mittel auf, die dafür eingerichtet sind, die erste Gasfraktion zumindest zum Teil einer zweiten Tieftemperaturrektifikation unter Verwendung eines flüssigen Rücklaufs, der überwiegend oder ausschließlich den Kohlenwasserstoff mit N - 1 Kohlenstoffatomen enthält, zu unterwerfen, in der die erste Gasfraktion an dem Olefin mit N Kohlenstoffatomen abgereichert wird.

Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

Kurze Beschreibung der Zeichnungen
Figur 1 veranschaulicht eine Anlage zur Herstellung von Olefinen gemäß einer Ausführungsform der Erfindung.
Figur 2 veranschaulicht eine Tieftemperaturtrennung zum Einsatz in einer Anlage zur Herstellung von Olefinen gemäß einer nicht erfindungsgemäßen Variante.
Figur 3 veranschaulicht eine Tieftemperaturtrennung zum Einsatz in einer Anlage Herstellung von Olefinen gemäß einer Ausführungsform der Erfindung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe oder eine nichtoxidative Dehydrierung. In diesem Fall gelten die nachfolgenden Erläuterungen entsprechend.

In der Anlage 100 wird einer Rektifikationseinheit 101 mit beispielsweise einer oder mehreren Rektifikationskolonnen ein Trenneinsatz in Form eines Stoffstroms a zugeführt und einer Rektifikation unterworfen. Der Trenneinsatz enthält im dargestellten Beispiel zumindest Ethan und höhere Kohlenwasserstoffe, insbesondere entsprechende höhere Paraffine. Der Rektifikationseinheit 101 können auch ein oder mehrere weitere Trenneinsätze zugeführt werden.

In der Rektifikationseinheit 101 wird der Trenneinsatz alleine oder zusammen mit dem oder den weiteren Trenneinsätzen unter Erhalt eines Trennprodukts, das Ethan enthält, aber arm an höheren Kohlenwasserstoffen ist, einer Rektifikation unterworfen. Das Trennprodukt wird in Form eines Stoffstroms c abgezogen und einer Vorwärmeinheit 102 zugeführt. In der Vorwärmeinheit 102 wird das Trennprodukt vorgewärmt, wobei der Vorwärmeinheit 102 im dargestellten Beispiel auch ein Wasser- oder Dampfstrom d zugeführt wird. Auch weitere Stoffströme können zugeführt werden. Zu dem Stoffstrom c kann ein weiterer, unten erläuterter Stoffstrom b zugespeist werden.

Ein aus der Vorwärmeinheit 102 abströmender Stoffstrom e wird zur Bildung eines Reaktionseinsatzes einer Reaktionseinheit 103 zugeführt. Der Reaktionseinsatz enthält aufgrund seiner Bildung unter Verwendung des Trennprodukts aus der Rektifikationseinheit 101 Ethan, ist aber arm an höheren Kohlenwasserstoffen. Der Reaktionseinsatz kann ferner ein oder mehrere Verdünnungsmittel wie Wasser oder Inertgase und weitere Komponenten enthalten. Diese können der Reaktionseinheit 103 auch in Form weiterer, nicht dargestellter Stoffströme zugeführt werden.

Der Reaktionseinheit 103 wird im dargestellten Beispiel ein sauerstoffhaltiger Stoffstrom f zugeführt. Dieser kann unter Verwendung einer Luftzerlegungsanlage 104 bereitgestellt werden. Der Luftzerlegungsanlage 104 wird hierzu ein Luftstrom g zugeführt. Bei dem sauerstoffhaltigen Stoffstrom f kann es sich um im Wesentlichen reinen Sauerstoff handeln, je nach Betrieb der Luftzerlegungsanlage 104 können jedoch auch Anteile an Stickstoff und an Edelgasen enthalten sein. Auf diese Weise ist es ebenfalls möglich, Verdünnungsmittel zuzuführen.

Aus der Reaktionseinheit 103 strömt ein Prozessgas in Form eines Prozessgasstroms h ab, in dem Ethylen enthalten ist, das in der Reaktionseinheit 103 durch ODH eines Teils des Ethans in dem Reaktionseinsatz gebildet wurden. Ferner enthält das Produktgemisch Essigsäure, die ebenfalls bei der ODH in der Reaktionseinheit 103 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in der Reaktionseinheit 103 gebildet.

Es versteht sich, dass Reaktionseinheit 103 einen, aber auch mehrere, beispielsweise parallel betriebene, Reaktoren aufweisen kann. In letzterem Fall werden diesen Reaktoren jeweils entsprechende Reaktionseinsätze, die gleich oder unterschiedlich zusammengesetzt sein können, sowie entsprechende sauerstoffhaltige Stoffströme f zugeführt werden, und es werden jeweils entsprechende Prozessgasströme h gebildet. Letztere können beispielsweise vereinigt und gemeinsam als Prozessgas den nachfolgend erläuterten Einheiten zugeführt werden.

Das Prozessgas wird in eine Quencheinheit 104 überführt, in der es, beispielsweise in einer Waschsäule, mit Waschwasser oder einer geeigneten wässrigen Lösung in Kontakt gebracht werden kann. In der Quencheinheit 104 wird das Prozessgas insbesondere abgekühlt und die in der Reaktionseinheit 103 gebildete Essigsäure wird aus dem Prozessgas ausgewaschen. Mit Essigsäure beladenes Prozesswasser strömt in Form eines Stoffstroms i aus der Quencheinheit 104 ab, das zumindest weitgehend von Essigsäure befreite Prozessgas in Form eines Stoffstroms k.

In einer optionalen Essigsäurerückgewinnungseinheit 105 wird aus dem mit Essigsäure beladenen Prozesswasser Essigsäure als Eisessig abgetrennt, welcher als Stoffstrom I aus der Anlage 100 ausgeführt wird. In der Essigsäurerückgewinnungseinheit 105 ebenfalls rückgewonnenes reines Prozesswasser kann in Form des bereits erläuterten Stoffstroms d der Vorwärmeinheit 102 zugeführt werden. Das dem Reaktor zugeführte Prozesswasser kann auch teilweise oder vollständig in Form von extern zugeführtem Frischwasser bereitgestellt werden. Nicht mehr brauchbares oder benötigtes Wasser kann in Form eines Abwasserstroms m aus der Anlage 100 ausgeführt und einer Abwasseraufbereitung zugeführt werden.

Das in Form des Stoffstroms k vorliegende, zumindest weitgehend von Essigsäure befreite Prozessgas wird in einer Verdichtungseinheit 106 auf ein geeignetes Druckniveau, beispielsweise 15 bis 25 bar, verdichtet und in Form eines verdichteten Stoffstroms n einer Aminwäscheeinheit 107 zugeführt. In dieser werden insbesondere Teile des in dem Prozessgas enthaltenen Kohlendioxids ausgewaschen. Nach Regenerierung des Amins kann das ausgewaschene Kohlendioxid in Form eines Stoffstroms q aus der Anlage ausgeführt werden.

Das derart teilweise von Kohlendioxid befreite Prozessgas wird in Form eines Stoffstroms o in eine Laugenwäscheeinheit 108 überführt und dort weiter von Kohlendioxid gereinigt. In der Laugenwäscheeinheit 108 fällt Ablauge an, die in Form eines Stoffstroms p in eine Ablaugebehandlungseinheit 109 überführt und schließlich aus der Anlage ausgeführt wird.

Das in der Laugenwäscheeinheit 108 weiter gereinigte Prozessgas wird in Form eines Stoffstroms r in eine Vorkühl- und Trockeneinheit 110 überführt, wo es insbesondere von Restwasser befreit werden kann. Das getrocknete Prozessgas wird in Form eines Stoffstroms s in eine Tieftemperatureinheit 111 überführt und anschließend in weiter abgekühlter Form in Form eines oder mehrerer Stoffströme t in eine Demethanisierungseinheit 112. In der Tieftemperatureinheit 111 und der Demethanisierungseinheit 112 werden tiefer als Ethylen siedende Komponenten, darunter insbesondere Kohlenmonoxid und Sauerstoff, aus dem Prozessgas abgetrennt, wobei der Rest in kondensierter Form verbleibt. Sind in dem Prozessgas höhere Kohlenwasserstoffe enthalten, die bei der ODH in der Reaktionseinheit 103 als Nebenprodukt gebildet wurden, gehen diese ebenfalls in das Kondensat über.

Die abgetrennten, tiefer als Ethylen siedenden Komponenten werden in Form eines oder mehrerer Stoffströme u durch die Tieftemperatureinheit 111 und die Vorkühl- und Trockeneinheit 110 zurückgeführt, dort ggf. mit weiteren entsprechender Stoffströme vereinigt, zu Kühlzwecken genutzt, und aus der Anlage 100 ausgeführt. Bei Bedarf werden die Kohlenwasserstoffe mit zwei und ggf. mehr Kohlenstoffatomen in Form eines Stoffstroms v einer Hydriereinheit 113 zugeführt, in der insbesondere Acetylen, das ebenfalls bei der ODH in der in der Reaktionseinheit 103 als Nebenprodukt gebildet wurde, hydriert werden kann. Nach der Hydrierung wird der nun mit w bezeichnete Stoffstrom in eine Ethylenabtrenneinheit 114 überführt.

In der Ethylenabtrenneinheit 114 wird Ethylen zumindest weitgehend von anderen Komponenten abgetrennt und kann in Form eines Stoffstroms x nach Nutzung in einer Ethylenkälteeinheit 115 gasförmig aus der Anlage 100 ausgeführt werden. Die übrigen Komponenten, überwiegend Ethan und ggf. höhere Kohlenwasserstoffe, werden in Form eines Stoffstroms y abgezogen und in Form des Stoffstroms b in die Vorwärmeinheit 102 zurückgeführt.

In die Demethanisierungseinheit 112 kann in der veranschaulichten erfindungsgemäßen Ausführungsform ein flüssiger methanreicher Strom z eingespeist werden, wie nachfolgend im Detail erläutert.

Figur 2 veranschaulicht eine Tieftemperaturtrenung zum Einsatz in einer Anlage zur Herstellung von Olefinen gemäß einer nicht erfindungsgemäßen Variante. Diese Tieftemperaturtrennung umfasst die Verwendung einer Tieftemperatureinheit und eine Demethanisierungseinheit, die in einer nicht erfindungsgemäßen Variante einer Anlage 100, wie sie in Figur 1 gezeigt ist, und in der keine Methaneinspeisung in Form des methanreichens Stroms z vorgenommen wird, zum Einsatz kommen können. Die Tieftemperatureinheit und eine Demethanisierungseinheit sind daher hier mit 111' und 112' zusammengefasst. Die bereits in Figur 1 dargestellten Stoffströme s, t, u und v sind auch hier gezeigt. Die Darstellung der jeweiligen Elemente ist nicht positions- und nicht maßstabsgerecht.

Das Prozessgas wird in Form des Stoffstroms s der Tieftemperatureinheit 111' zugeführt. Das Prozessgas wird nacheinander durch Wärmetauscher 201 bis 204 geführt und dort auf immer niedrigere Temperaturniveaus gekühlt. Die Wärmetauscher 201 bis 204 können dazu mit nicht veranschaulichten Ethylenströmen gekühlt werden. Zur Kühlung kann, wie ebenfalls nicht gesondert veranschaulicht, zusätzlich der Stoffstrom u eingesetzt werden, der im dargestellten Beispiel die tiefer als Ethan siedenden Komponenten des Prozessgases enthält, die in der Tieftemperatureinheit 111' und der Demethanisierungseinheit 112' abgetrennt werden.

Stromab der Wärmetauscher 201 bis 204 wird das Prozessgas bzw. ein durch die Abkühlung in den Wärmetauschern 201 bis 204 jeweils gebildetes Zweiphasengemisch jeweils in Abscheider 205 bis 208 überführt, wo sich jeweils ein Kondensat aus dem Prozessgas abscheidet. Die Kondensate werden in einer ihrer stofflichen Zusammensetzung entsprechenden Höhe in Form der Stoffströme t in eine Rektifikationskolonne 209 der Demethanisierungseinheit 112, den sogenannten Demethanizer, eingespeist. Ein geringerer Anteil des Prozessgases kann auch, wie hier nicht veranschaulicht, direkt in die Rektifikationskolonne 209 eingespeist werden.

Ein Sumpfverdampfer 210 der Rektifikationskolonne 209 wird beispielsweise unter Verwendung von Propan oder Propylen aus einem Kältekreislauf beheizt, ein Kopfkondensator 211 beispielsweise unter Verwendung von Niederdruckethylen gekühlt. Die Rektifikationskolonne 209 wird derart betrieben, dass sich an ihrem Kopf überwiegend Methan und tiefer siedende Komponenten und in ihrem Sumpf die höher siedenden Verbindungen anreichern. Auf diese Weise kann vom Kopf der Rektifikationskolonne 209 ein Teil des Stoffstroms u, hier mit u1 bezeichnet, und aus dem Sumpf der Rektifikationskolonne 7 der Stoffstrom v abgezogen werden. Ein in dem Abscheider 208 gasförmig verbliebener Anteil des Prozessgases, hier in Form eines Stoffstroms u2 veranschaulicht, kann ebenfalls bei der Bildung des Stoffstroms u verwendet werden.

In einem repräsentativen Beispiel werden beispielsweise ca. 67 t/h an Prozessgas in Form des Stoffstroms s der Tieftemperatureinheit 111' auf einem Druck von beispielsweise ca. 20 bar zugeführt. Die Temperatur stromab des Wärmetauschers 201 beträgt beispielsweise ca. -30 °C, die Temperatur stromab des Wärmetauschers 202 beispielsweise ca. -50 °C, die Temperatur strom ab des Wärmetauschers 203 beispielsweise ca. -75 °C und die Temperatur stromab des Wärmetauschers 204 beispielsweise ca. -99 °C. Der Sumpfverdampfer 210 wird auf einem Temperaturniveau von beispielsweise ca. -17 °C, der Kopfkondensator 211 auf einem Temperaturniveau von beispielsweise ca. -97 °C betrieben.

Der Stoffstrom u1 umfasst in diesem Beispiel einen Mengenstrom von insgesamt beispielsweise ca. 2 t/h, davon beispielsweise ca. 190 kg/h Ethylen und beispielsweise ca. 13 mol-% Sauerstoff. Der Stoffstrom u2 umfasst einen Mengenstrom von insgesamt beispielsweise ca. 1 t/h, davon beispielsweise ca. 40 kg/h Ethylen und beispielsweise ca. 13 mol-% Sauerstoff.

Man erkennt damit, dass in Form der Stoffströme u1 und u2 bzw. des Stoffstroms u beträchtliche Mengen an Ethylenprodukt verloren gehen, die eigentlich in den Stoffstrom v überführt werden sollten. Dies ist, wie erwähnt, insbesondere auf die vergleichsweise geringen Methangehalte in dem Prozessgas des Stoffstroms s zurückzuführen, die bei den angegebenen Bedingungen keine befriedigende Trennung in der Rektifikationskolonne 209 zulassen. Gleichzeitig ist die hohe Sauerstoffkonzentration, insbesondere in Anbetracht des ebenfalls vorhandenen Kohlenmonoxids, kritisch.

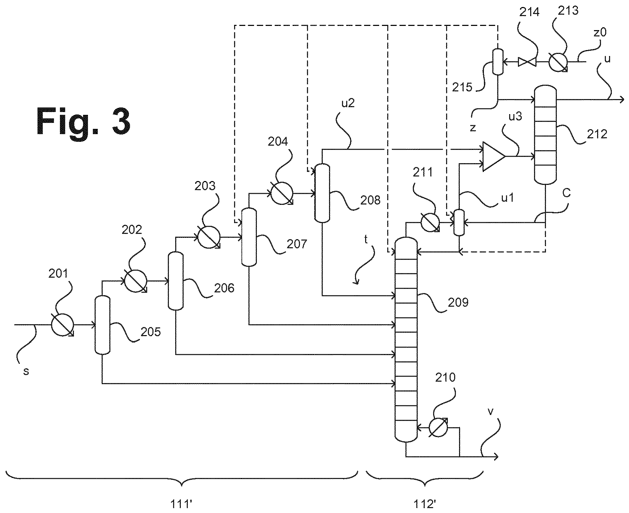

Figur 3 veranschaulicht eine Tieftemperaturtrennung zum Einsatz in einer Anlage zur Herstellung von Olefinen gemäß einer Ausführungsform der Erfindung, in der diese Probleme behoben sind. Auch diese Tieftemperaturtrennung umfasst die Verwendung einer Tieftemperatureinheit und einer Demethanisierungseinheit, die beispielsweise in einer erfindungsgemäßen Anlage 100, wie sie in Figur 1 gezeigt ist, zum Einsatz kommen können. Die Tieftemperatureinheit und eine Demethanisierungseinheit sind daher hier mit 111 und 112 zusammengefasst. Die bereits in Figur 1 dargestellten Stoffströme s, t, u und v sind auch hier gezeigt. Die Darstellung der jeweiligen Elemente ist nicht positions- und nicht maßstabsgerecht. Auf die bereits zu Figur 3 erfolgten Erläuterungen betreffend die Demethanisierungseinheit 111' und 112' wird Bezug genommen, soweit nachfolgend nichts Anderes angegeben ist.

Im Gegensatz zu der in der Figur 2 veranschaulichten Tieftemperaturtrennung wird in der in Figur 3 veranschaulichten Tieftemperaturtrennung ein durch Vereinigen der Stoffströme u1 und u2 gebildeter Stoffstrom noch nicht direkt in Form des Stoffstroms u abgezogen, sondern dieser, hier mit u3 bezeichnet, wird in eine zweite Rektifikationskolonne 212 eingespeist. Am Kopf der zweiten Rektifikationskolonne 212 wird in der Form eines Rücklaufs der flüssige, methanreiche Strom z aufgegeben.

Auf diese Weise kann der Massenstrom an Ethylen in dem Stoffstrom u bei einem Massenstrom von beispielsweise ca. 3 bis 4 t/h insgesamt auf beispielsweise nurmehr ca. 5 kg/h abgesenkt werden. Der Stoffstrom u enthält gleichzeitig beispielsweise nurmehr ca. 6 mol-% Sauerstoff, ist also diesbezüglich als unkritisch anzusehen.

Zur Bereitstellung des methanreichen Rücklaufs auf die zweite Rektifikationskolonne 212 wird ein verdichteter methanreicher Stoffstrom z0 von beispielsweise ca. 1,5 t/h auf einem Druckniveau von beispielsweise ca. 40 bar bereitgestellt und in einem Wärmetauscher 213 auf ein Temperaturniveau von beispielsweise ca. - 90°C gekühlt. Nach einer Entspannung in einem Ventil 214 liegt ein Zweiphasengemisch vor, das in einen Abscheider 215 eingespeist und dort phasengetrennt wird.

Die Flüssigphase wird im dargestellten Beispiel vollständig in die zweite Rektifikationskolonne 212 eingespeist, die Gasphase kann jeweils zum Teil oder vollständig in die Abscheidebehälter 205, 206, 207 und 208, die Rektifikationskolonne 209 und einen dem Kopfkondensator 211 nachgeschalteten Abscheider eingespeist werden. Alle diese Einspeisungen der Gasphase sind optional und alternativ zueinander möglich und daher mit gestrichelten Pfeilen veranschaulicht. Sie dienen insbesondere dazu, einen kritischen Sauerstoffgehalt in den genannten Einheiten zu reduzieren.

Im Sumpf der zweiten Rektifikationskolonne 212 wird eine Flüssigkeit gewonnen, die insbesondere das unter Verwendung des flüssigen Rücklaufs rückgewonnene Ethylen enthält und ansonsten methanreich ist. Diese kann in Form eines Stoffstroms C in den dem Kopfkondensator 211 nachgeschalteten Abscheider eingespeist oder alternativ dazu, wie mit einem gestrichelten Pfeil veranschaulicht, direkt in die Rektifikationskolonne 209 zurückgeführt werden.

## Patentansprüche

1. Verfahren zur Herstellung eines Olefins mit N Kohlenstoffatomen, bei dem unter Einsatz einer Dehydrierung ein Prozessgas gebildet wird, das zumindest das Olefin mit N Kohlenstoffatomen, ein Paraffin mit N Kohlenstoffatomen und einen Kohlenwasserstoff mit N - 1 Kohlenstoffatomen enthält, und bei dem unter Verwendung zumindest eines Teils des Prozessgases ein Trenneinsatz gebildet wird, der einer Tieftemperaturtrennung unterworfen wird, in welcher der Trenneinsatz über mehrere Temperaturniveaus stufenweise abgekühlt und Kondensate aus dem Trenneinsatz abgeschieden werden, wobei die Kondensate zumindest zum Teil unter Erhalt einer ersten Gasfraktion und einer ersten Flüssigfraktion einer ersten Tieftemperaturrektifikation unterworfen werden, wobei die erste Gasfraktion zumindest das Olefin mit N Kohlenstoffatomen in einem geringeren Anteil als in den Kondensaten und den Kohlenwasserstoff mit N - 1 Kohlenstoffatomen in einem höheren Anteil als in den Kondensaten enthält, **dadurch gekennzeichnet, dass** die erste Gasfraktion zumindest zum Teil einer zweiten Tieftemperaturrektifikation unter Verwendung eines flüssigen Rücklaufs, der überwiegend oder ausschließlich den Kohlenwasserstoff mit N - 1 Kohlenstoffatomen enthält, unterworfen wird, in der die erste Gasfraktion an dem Olefin mit N Kohlenstoffatomen abgereichert wird.

2. Verfahren nach Anspruch 1, bei dem ein bei der stufenweisen Abkühlung gasförmig verbleibender Anteil des Trenneinsatzes ebenfalls zumindest teilweise der zweiten Tieftemperaturrektifikation unterworfen wird.

3. Verfahren nach Anspruch 1 oder 2, bei der in der zweiten Tieftemperaturrektifikation eine zweite Gasfraktion, die arm an oder frei von dem Olefin mit N Kohlenstoffatomen ist, und eine zweite Flüssigfraktion, die das Olefin mit N Kohlenstoffatomen in einem höheren Anteil als in der ersten Gasfraktion enthält, gebildet werden.

4. Verfahren nach Anspruch 3, bei dem die zweite Flüssigfraktion in die erste Tieftemperaturrektifikation zurückgeführt wird.

5. Verfahren nach einem der vorstehenden Ansprüche, bei dem der flüssige Rücklauf als verflüssigtes Gas bereitgestellt wird, oder bei dem der flüssige Rücklauf unter Verwendung einer flüssigen Fraktion gebildet wird, die durch Teilkondensieren eines überwiegend oder ausschließlich den Kohlenwasserstoff mit N - 1 Kohlenstoffatomen enthaltenden Gases gebildet wird.

6. Verfahren nach Anspruch 5, bei dem ein bei dem Teilkondensieren des überwiegend oder ausschließlich den Kohlenwasserstoff mit N - 1 Kohlenstoffatomen enthaltenden Gases gasförmig verbleibender Anteil zumindest zum Teil in die Tieftemperaturtrennung eingespeist wird.

7. Verfahren nach Anspruch 5 oder 6, bei dem bei dem das verflüssigte Gas oder die flüssige Fraktion teilweise in die Tieftemperaturtrennung eingespeist wird.

8. Verfahren nach einem der vorstehenden Ansprüche, bei dem N zwei ist, so dass das Olefin mit N Kohlenstoffatomen Ethylen, das Paraffin mit N Kohlenstoffatomen Ethan und der Kohlenwasserstoff mit N - 1 Kohlenstoffatomen Methan ist.

9. Verfahren nach Anspruch 6, bei dem die Dehydrierung oxidativ durchgeführt wird.

10. Verfahren nach Anspruch 8 oder 9, bei dem die Temperaturniveaus, auf die der Trenneinsatz stufenweise abgekühlt wird, ein erstes Temperaturniveau von -20 bis -40 °C und/oder ein zweites Temperaturniveau von -40 bis -60 °C und/oder ein drittes Temperaturniveau von -70 bis -80 °C und /oder ein viertes Temperaturniveau von -95 bis -105 °C umfassen.

11. Verfahren nach einem der Ansprüche 8 bis 10, bei dem der flüssige Rücklauf, der überwiegend oder ausschließlich Methan enthält, in Form von verflüssigtem Erdgas bereitgestellt oder unter Verwendung zumindest eines Teils einer flüssigen Fraktion gebildet wird, die durch Abkühlen eines überwiegend oder ausschließlich Methan enthaltenden Gases auf einem Druckniveau von 30 bis 70 bar auf ein Temperaturniveau von -70 bis -100 °C und Entspannen auf ein Druckniveau von 10 bis 20 bar bereitgestellt wird.

12. Verfahren nach einem der vorstehenden Ansprüche, bei dem für die zweite Tieftemperaturrektifikation eine Rektifikationskolonne mit 1 bis 10 theoretischen oder praktischen Böden eingesetzt wird.

13. Verfahren nach einem der vorstehenden Ansprüche, bei dem das Prozessgas ferner Sauerstoff und Kohlenmonoxid enthält.

14. Anlage (100) zur Herstellung eines Olefins mit N Kohlenstoffatomen, mit wenigstens einer Reaktoreinheit (103), die dafür eingerichtet ist, unter Einsatz einer Dehydrierung ein Prozessgas zu bilden, das zumindest das Olefin mit N Kohlenstoffatomen, ein Paraffin mit N Kohlenstoffatomen und einen Kohlenwasserstoff mit N - 1 Kohlenstoffatomen enthält, und mit Mitteln, die dafür eingerichtet sind, unter Verwendung zumindest eines Teils des Prozessgases ein Trenneinsatz zu bilden und einer Tieftemperaturtrennung zu unterwerfen, die dafür eingerichtet ist, den Trenneinsatz über mehrere Temperaturniveaus stufenweise abzukühlen und Kondensate aus dem Trenneinsatz abzuscheiden sowie die Kondensate zumindest zum Teil unter Erhalt einer ersten Gasfraktion und einer ersten Flüssigfraktion einer ersten Tieftemperaturrektifikation zu unterwerfen, wobei die erste Gasfraktion zumindest das Olefin mit N Kohlenstoffatomen in einem geringeren Anteil als in den Kondensaten und den Kohlenwasserstoff mit N - 1 Kohlenstoffatomen in einem höheren Anteil als in den Kondensaten enthält, **dadurch gekennzeichnet, dass** Mittel vorgesehen sind, die dafür eingerichtet sind, die erste Gasfraktion zumindest zum Teil einer zweiten Tieftemperaturrektifikation unter Verwendung eines flüssigen Rücklaufs, der überwiegend oder ausschließlich den Kohlenwasserstoff mit N - 1 Kohlenstoffatomen enthält, zu unterwerfen, in der die erste Gasfraktion an dem Olefin mit N Kohlenstoffatomen abgereichert wird.

15. Anlage (100) nach Anspruch 14, die zur Durchführung eines Verfahrens nach einem der Ansprüche 1 bis 13 eingerichtet ist.

## Claims

1. Method for manufacturing an olefin having N carbon atoms, wherein a process gas is formed using dehydrogenation, which gas contains at least the olefin having N carbon atoms, a paraffin having N carbon atoms and a hydrocarbon having N - 1 carbon atoms, and wherein using at least one part of the process gas a fractional charge is formed, which is subjected to a cryogenic separation in which the fractional charge is cooled gradually across a plurality of temperature levels, and condensates from the fractional charge are deposited, wherein the condensates are subjected at least in part to a first cryogenic rectification with a first gas fraction and a first liquid fraction being obtained, wherein the first gas fraction contains at least the olefin having N carbon atoms in a smaller proportion than in the condensates and the hydrocarbon having N - 1 carbon atoms in a higher proportion than in the condensates, **characterized in that** the first gas fraction is subjected at least in part to a second cryogenic rectification, using a liquid reflux, which predominantly or exclusively contains the hydrocarbon having N - 1 carbon atoms, in which the first gas fraction is depleted of the olefin having N carbon atoms.

2. Method according to claim 1, wherein a portion of the fractional charge which remains gaseous during the gradual cooling is likewise at least partially subjected to the second cryogenic rectification.

3. Method according to claim 1 or 2, wherein in the second cryogenic rectification, a second gas fraction, which is low in or free from the olefin having N carbon atoms, and a second liquid fraction containing the olefin having N carbon atoms in a higher proportion than in the first gas fraction, are formed.

4. Method according to claim 3, wherein the second liquid fraction is recycled to the first cryogenic rectification.

5. Method according to any one of the preceding claims, wherein the liquid reflux is provided as liquefied gas or wherein the liquid reflux is formed using a liquid fraction formed by partial condensation of a gas containing predominantly or exclusively the hydrocarbon having N - 1 carbon atoms.

6. Method according to claim 5, wherein a portion, which remains gaseous in the partial condensation of the gas containing predominantly or exclusively the hydrocarbon having N - 1 carbon atoms, is at least in part fed into the cryogenic separation.

7. Method according to claim 5 or 6, wherein the liquefied gas or the liquid fraction is fed in part into the cryogenic separation.

8. Method according to any one of the preceding claims, wherein N is two so that the olefin having N carbon atoms is ethylene, the paraffin having N carbon atoms is ethane, and the carbon having N - 1 hydrocarbon atoms is methane.

9. Method according to claim 6, wherein the dehydrogenation is carried out oxidatively.

10. Method according to claim 8 or 9, wherein the temperature levels to which the fractional charge is gradually cooled comprise a first temperature level of from -20 to -40°C and/or a second temperature level of from -40 to -60°C and/or a third temperature level of from -70 to -80°C and/or a fourth temperature level of from -95 to -105°C.

11. Method according to any one of claims 8 to 10, wherein the liquid reflux, which predominantly or exclusively contains methane, is provided in the form of liquefied natural gas or formed using at least a portion of a liquid fraction provided by cooling a gas containing predominantly or exclusively methane at a pressure level of from 30 to 70 bar to a temperature level of from -70 to -100°C and depressurizing to a pressure level of from 10 to 20 bar.

12. Method according to any one of the preceding claims, wherein a rectification column having from 1 to 10 theoretical or practical trays is used for the second cryogenic rectification.

13. Method according to any one of the preceding claims, wherein the process gas further contains oxygen and carbon monoxide.

14. Assembly (100) for manufacturing an olefin having N carbon atoms with at least one reactor unit (103) configured to form a process gas using dehydrogenation, which gas contains at least the olefin having N carbon atoms, a paraffin having N carbon atoms and a hydrocarbon having N - 1 carbon atoms, and with means configured to form a fractional charge using at least one part of the process gas and to subject it to a cryogenic separation which is configured to cool the fractional charge gradually across a plurality of temperature levels and to deposit condensates from the fractional charge and to subject the condensates at least in part to a first cryogenic rectification with a first gas fraction and a first liquid fraction being obtained, wherein the first gas fraction contains at least the olefin having N carbon atoms in a smaller proportion than in the condensates and the hydrocarbon having N - 1 carbon atoms in a higher proportion than in the condensates, **characterized in that** means are provided which are configured to subject the first gas fraction at least in part to a second cryogenic rectification, using a liquid reflux which predominantly or exclusively contains the hydrocarbon having N - 1 carbon atoms, in which the first gas fraction is depleted of the olefin having N carbon atoms.

15. Assembly (100) according to claim 14, configured to carry out a method according to any one of claims 1 to 13.

## Revendications

1. Procédé de production d'une oléfine ayant N atomes de carbone, selon lequel, au moyen d'une déshydrogénation, un gaz de processus est formé, lequel contient au moins l'oléfine ayant N atomes de carbone, une paraffine ayant N atomes de carbone et un hydrocarbure ayant N - 1 atomes de carbone, et selon lequel, à l'aide d'au moins une partie du gaz de processus, une charge de séparation, laquelle est soumise à une séparation à basse température, lors de laquelle la charge de séparation est refroidie par étapes en passant par plusieurs niveaux de températures, et des condensats sont séparés de la charge de séparation, dans lequel les condensats sont, au moins partiellement, et pour obtenir une première fraction gazeuse et une première fraction liquide, soumis à une première rectification à basse température, dans lequel la première fraction gazeuse contient au moins l'oléfine ayant N atomes de carbone dans une proportion inférieure aux condensats, et l'hydrocarbure ayant N - 1 atomes de carbone dans une proportion supérieure aux condensats, **caractérisé en ce que** la première fraction gazeuse est, au moins partiellement, soumise à une seconde rectification à basse température à l'aide d'un reflux liquide, lequel contient principalement ou exclusivement l'hydrocarbure ayant N - 1 atomes de carbone, lors de laquelle la première fraction gazeuse est appauvrie en l'oléfine ayant N atomes de carbone.

2. Procédé selon la revendication 1, selon lequel une proportion de la charge de séparation restante gazeuse lors du refroidissement par étapes est également soumise, au moins partiellement, à la seconde rectification à basse température.

3. Procédé selon la revendication 1 ou 2, selon lequel, dans la seconde rectification à basse température, une seconde fraction gazeuse, laquelle contient peu ou pas d'oléfine ayant N atomes de carbone, et une seconde fraction liquide, laquelle contient l'oléfine ayant N atomes de carbone dans une proportion supérieure à la première fraction gazeuse, sont formées.

4. Procédé selon la revendication 3, selon lequel la seconde fraction liquide est renvoyée à la première rectification à basse température.

5. Procédé selon l'une quelconque des revendications précédentes, selon lequel le reflux liquide est fourni sous forme de gaz liquéfié ou selon lequel le reflux liquide est formé à l'aide d'une fraction liquide obtenue par condensation partielle d'un gaz contenant principalement ou exclusivement l'hydrocarbure ayant N - 1 atomes de carbone.

6. Procédé selon la revendication 5, dans lequel une proportion restante gazeuse lors de la condensation partielle du gaz contenant principalement ou exclusivement l'hydrocarbure ayant N-1 atomes de carbone est injectée au moins partiellement dans la séparation à basse température.

7. Procédé selon la revendication 5 ou 6, selon lequel le gaz liquéfié ou la fraction liquide est partiellement injecté dans la séparation à basse température.

8. Procédé selon l'une quelconque des revendications précédentes dans lequel N est deux de telle sorte que l'oléfine ayant N atomes de carbone est l'éthylène, la paraffine ayant N atomes de carbone est l'éthane et l'hydrocarbure ayant N - 1 atomes de carbone est le méthane.

9. Procédé selon la revendication 6, selon lequel la déshydrogénation est effectuée par voie oxydative.

10. Procédé selon la revendication 8 ou 9, selon lequel les niveaux de température auxquels la charge de séparation est refroidie par étapes comprennent un premier niveau de température allant de -20 à -40 °C et/ou un deuxième niveau de température allant de -40 à -60 °C et/ou un troisième niveau de température allant de -70 à -80 °C et/ou un quatrième niveau de température allant de -95 à - 105 °C.

11. Procédé selon l'une quelconque des revendications 8 à 10, selon lequel le reflux liquide, lequel contient principalement ou exclusivement du méthane est fourni sous la forme de gaz naturel liquéfié ou est formé à l'aide d'au moins une partie d'une fraction liquide, laquelle est obtenue par refroidissement d'un gaz contenant principalement ou exclusivement du méthane à un niveau de pression allant de 30 à 70 bars jusqu'à un niveau de température allant de -70 à -100 °C et par dépressurisation à un niveau de pression allant de 10 à 20 bars.

12. Procédé selon l'une quelconque des revendications précédentes, selon lequel, pour la seconde rectification à basse température, une colonne de rectification comportant 1 à 10 plateaux théoriques ou pratiques est utilisée.

13. Procédé selon l'une quelconque des revendications précédentes, dans lequel le gaz de processus contient également de l'oxygène et du monoxyde de carbone.

14. Installation (100) de production d'une oléfine ayant N atomes de carbone, comprenant au moins une unité de réacteur (103), laquelle est conçue pour former un gaz de processus à l'aide d'une déshydratation, lequel contient au moins l'oléfine ayant N atomes de carbone, une paraffine ayant N atomes de carbone et un hydrocarbure ayant N - 1 atomes de carbone, et, des moyens, lesquels sont conçus pour, à l'aide d'au moins une partie du gaz de processus, former une charge de séparation pour la soumettre à une séparation à basse température, laquelle est conçue pour refroidir la charge de séparation par étapes en passant par plusieurs niveaux de températures et pour séparer les condensats de la charge de séparation, ainsi que pour soumettre les condensats, au moins partiellement, pour obtenir une première fraction gazeuse et une première fraction liquide, à une première rectification à basse température, dans laquelle la première fraction gazeuse contient au moins l'oléfine ayant N atomes de carbone dans une proportion inférieure aux condensats et l'hydrocarbure avec N - 1 atomes de carbone dans une proportion supérieure aux condensats, **caractérisée en ce que,** des moyens sont conçus pour soumettre la première fraction gazeuse au moins partiellement à une seconde rectification à basse température à l'aide d'un reflux liquide, lequel contient principalement ou exclusivement l'hydrocarbure avec N - 1 atomes de carbone, lors de laquelle la première fraction gazeuse est appauvrie en oléfine à N atomes de carbone.

15. Installation (100) selon la revendication 14, laquelle est conçue pour mettre en œuvre un procédé selon l'une quelconque des revendications 1 à 13.
